# NEUE EUROPÄISCHE PATENTSCHRIFT

(11) **EP 1 941 917 B2**
(45) Veröffentlichungstag und Bekanntmachung des Hinweises auf die Entscheidung über den Einspruch: **24.01.2024**
(45) Hinweis auf die Patenterteilung: 19.04.2017
(21) Anmeldenummer: 07023001.6
(22) Anmeldetag: 28.11.2007
(51) Int. Cl.: A61L 29/12

(54) **Katheterrohrelement**
Catheter pipe element
Elément de tuyau de cathéter

(30) Priorität: 22.12.2006 DE 102006062187
(43) Veröffentlichungstag der Anmeldung: 09.07.2008
(73) Patentinhaber: BIOTRONIK AG, 8180 Bülach (CH)
(72) Erfinder: Gielenz, Gerhard, 8620 Wetzikon (CH); Hofmann, Eugen, 8049 Zürich (CH); Surber, Bettina, 8600 Dübendorf (CH); Wolfer, Markus, 8800 Thalwill (CH)
(74) Vertreter: Keil & Schaafhausen Patentanwälte PartGmbB

(56) Entgegenhaltungen:
- EP-A1- 1 839 686
- FR-A1- 2 444 063
- US-A1- 2006 198 976
- MINOSIO J-P ET AL: "Lack of chemical reactivity between short catheters (ETFE) and three major antibiotics. Study model" ANNALES PHARMACEUTIQUES FRANCAISES, MASSON, PARIS, FR, Bd. 52, Nr. 6, 1. Januar 1994 (1994-01-01) , Seiten 303-310, XP008102275 ISSN: 0003-4509

## Beschreibung

Die Erfindung betrifft ein mehrschichtiges Katheterrohrelement, vorzugsweise zur Anordnung eines Führungsdrahtes, mit einer die äußere Schicht bildenden ersten Schicht und mindestens teilweise die innere Schicht bildenden zweiten Schicht. Die Erfindung betrifft ferner ein Verfahren zur Herstellung eines derartigen Katheterrohrelements.

Als Katheter werden Röhrchen oder Schläuche verschiedenen Durchmessers aus Materialien wie Kunststoff, Latex, Silikon, Metall oder Glas bezeichnet, mit denen Hohlorgane wie Harnblase, Magen, Darm, Gefäße, das Ohr oder das Herz sondiert, entleert, gefüllt oder gespült werden können. So genannte interventionelle Katheter, die für Interventionen, wie z.B. eine perkutane transluminale Angioplastie, verwendet werden, weisen eine Durchgangsöffnung auf, die zur Aufnahme eines Führungsdrahtes bestimmt ist. Zur Erweiterung oder Wiederöffnung eines verengten oder verschlossenen Blutgefäßes wird ein Ballonkatheter, d.h. ein Katheter, der an seinem distalen Ende ein Ballonelement aufweist, entlang des Führungsdrahts eingeführt und an der Stelle des Gefäßes platziert, die geöffnet oder geweitet werden soll.

Im Folgenden wird als Katheterrohrelement ein Abschnitt eines Katheters bezeichnet, der sich mindestens über einen Teil der Länge des Katheters erstreckt. Vorzugsweise umfasst das Katheterrohrelement auch den Abschnitt eines Ballonkatheters, in dem der Ballon angeordnet ist.

Für die interventionelle Katheterisierung werden häufig Katheterrohrelemente verwendet, welche zwei übereinander angeordnete Schichten aufweisen, die aneinander befestigt sind, wobei diese Schichten in ihren mechanischen Eigenschaften unterschiedlich beschaffen sind.

Ein in EP 0 650 740 B1 beschriebener Katheter weist eine innere Schicht auf, welche die mittig angeordnete Durchgangsöffnung bildet und aus Polyethylen besteht. Die äußere Schicht besteht aus Polyamid und weist an ihrem distalen Ende den Ballon zur Dilatation auf, der an die äußere Polyamidschicht des Katheterrohres geschweißt ist. Derartige Schläuche haben den Nachteil, dass die Verbindung zwischen den Schichten schwach ist, so dass häufig Delaminationsprobleme auftreten. Der Führungsdraht in dem longitudinalen Lumen des Katheters sitzt häufig fest und wird deshalb beim Herausziehen des Ballons durch diesen mitgenommen. Dadurch muss der Führungsdraht für eine erneute Dilatation noch einmal in das Gefäß eingeschoben werden.

In WO 92/11893 A1 werden Katheterröhrchen beschrieben, die aus einer äußeren Schicht aus einem harten Kunststoffmaterial wie Polyethylen, Polypropylen, Polycarbonat, Polysulfonat, Polymethylmethacrylat oder Nylon bestehen und aus einer inneren Schicht aus einem weichen elastomeren Kunststoffmaterial wie PVC, Silikonharz, Polyurethan. Mit diesen Materialien kann ein haltbarer und flexibler Katheter hergestellt werden, der einen kleinen Knickradius und eine gute Federcharakteristik (Elastizität) aufweist. Nachteilig ist jedoch, dass die äußere Schicht schlecht schweißbar ist, so dass ein Ballon nicht oder nicht fest genug an dem Katheterröhrchen befestigt werden kann.

Die aus WO 91/17782 A1 bekannten Katheterröhrchen weisen zwei nebeneinander liegende Segmente auf. Das erste, flexible Segment besteht aus einem Low Density Polyethylen (LDPE) oder Silikon. Das zweite, steife Segment besteht aus Polypropylen oder einem High Density Polyethylen (HDPE). Das steife Segment nimmt 70% bis 90% der Gesamtlänge des Röhrchens ein, so dass auf diese Weise ein für viele Anwendungen in diesem Bereich zu steifes Katheterrohrelement vorliegt.

Die US 2006/0198976 A1 beschreibt einen mehrlumigen Katheter, der mit Hilfe eines Schrumpfschlauches hergestellt wird und der dreidimensionale Muster aufweist.

In der französichen Patentschrift mit der Veröffentlichungsnummer N° 2 444 063 wird ein Schlauch offenbart, der aus einem Ethylen-Vinylacetat-Copolymer besteht.

Weitere bekannte Katheterrohrelemente setzen sich aus einer äußeren Polyamidschicht und einer inneren Schicht aus Polytetrafluorethylen (PTFE) zusammen. Ein derartiges Katheterrohrelement weist sehr gute Gleiteigenschaften auf, ist jedoch nicht strahlensterilisierbar, da PTFE durch die bei der Strahlensterilisierung verwendete ionisierende Strahlung (Röntgen- oder Gammastrahlung, auch Elektronenbeschuss) geschädigt wird.

Die EP 1 839 686 A1 Katheter mit einer inneren Katheterschicht, die aus einer Zusammensetzung besteht, die ETFE und PTFE in einem Massenverhältnis von 99:1 bis 45:55 enthält. Weiterhn weisst dieser Katheder eine äußere Harzschicht auf.

Ein Katheder gemäß der WO 95/15780 besteht aus drei Schichten, wobei die innere Schicht aus einem Copolymer aus Polyvinylidenfluorid und Hexafluorpropylen und die äußere Schicht aus einer Mischung aus Nylon und Polyetherblockamid oder Polyetherblockamid allein gebildet ist. Zwischen innerer und äußerer Schicht findet sich die Verstarkungsschicht.

Aus der WO 2006/039392 A2 ist ein Katheder bekannt, der aus einem Innenrohr und einer um dises Innenrohr herum gebildeten mehrschichtige Hülle besteht. Die mehrschichtige Hülle beinhaltet eine Zwischenschicht, die von einem ersten Polyetherblockamidmaterial gebildet wird und eine von einem anderen zweiten Polyetherblockamid gebildete Außenschicht. Daziwschen kann Verstärkungsmaterial angeordnet sein.

Die Aufgabe der vorliegenden Erfindung besteht demnach darin, ein Katheterrohrelement zu schaffen, welches sehr gute Biegeeigenschaften und Gleiteigenschaften aufweist, an seiner äußeren Oberfläche schweißbar ist sowie eine Strahlensterilisierung ermöglicht.

Die Aufgabe wird durch ein Katheterrohrelement nach Anspruch 1 gelöst, dessen erste Schicht mindestens ein Kunststoff oder mehrere Kunststoffe aus der Gruppe Polyamid und Elastomer modif ziertes Polyamid wie z.B. Polyether-Block-Amid (PEBA) enthält und dessenzweite Schicht aus Ethylentetrafluorethylen (ETFE)besteht, dadurch gekennzeichnet,
dass das Katheterrohrelement mittels Koextrusion oder mittels eines Tauchverfahrens, das vorzugsweise mehrstufig ist, hergestellt ist.

Der Vorteil des erfindungsgemäßen Katheterelements besteht darin, dass die erste Schicht bestehend aus einem oder mehreren der angegebenen Materialien gut schweißbar ist, so dass ein Ballon auf das Katheterelement geschweißt werden kann. Zudem lässt sich eine gute Verbindung zwischen den Kuns stoffen der ersten Schicht und ETFE erzielen, so dass Delaminationsprobleme nicht auftreten. Die bei dem erfindungsgemä-ßen Katheterelement verwendeten Kunststoffe, insbesondere ETFE, sind außerdem strahlensterilisierbar. Ein weiterer Vorteil des Materials ETFE besteht darin, dass es gute Gleiteigenschaften besitzt, chemikalienbeständig ist und bei Temperaturen bis 150°C eingesetzt werden kann. ETFE ist ein sehr stabiles Fluorpolymer und kann in Schichtdicken bis zu 1000 mm (1 mm) aufgetragen werden. Das Material ist zudem elektrisch sehr gut isolierend und chemisch beständig gegen fast alle Medien. Außerdem ist ETFE im Gegensatz zu PTFE thermoplastisch umformbar.

Die innere Schicht muss nicht die gesamte Innenfläche des Katheters bedecken. In der einfachsten, vorteilhaftesten Ausführungsform ist das Katheterrohrelement hohlzylinderförmig ausgebildet, so dass der Querschnitt durch einen Kreisring gebildet wird. In weiteren Ausführungsbeispielen kann das Katheterrohrelement auch anders geformt sein, beispielsweise einen Querschnitt in Form eines Ellipsenrings, eines Rechteckrings oder eines anderen Vieleckrings mit abgerundeten Ecken aufweisen. Die durchgehende Öffnung des Katheterrohrelements ist vorzugsweise etwa mittig angeordnet. Ebenfalls möglich ist die Versetzung der durchgehenden Öffnung des Katheterrohrelements etwas aus der Mitte heraus.

Ebenfalls ein sehr einfacher Aufbau, der zu einem sehr kleinen, vorteilhaften Gesamtdurchmesser des Katheterrohrelements führt, besteht aus genau zwei, fest miteinander verbundenen Schichten. Für eine geeignte Flexibilität des Katheterrohrelements weist die erste Schicht einen Härtegrad zwischen etwa 45 bis etwa 72 Shore D auf. Das Katheterrohrelement ist einfach gestaltet, da die zweite Schicht vollständig aus ETFE besteht.

Um gute Gleiteigenschaften des Katheterrohrelements zu erreichen, ist es ausreichend, wenn die innen liegende, zweite Schicht nur dünn mit einer Schichtdicke von wenigen Mikrometern (µm) ausgebildet ist und vorzugsweise eine Schichtdicke von mindestens 5 µm, besonders bevorzugt von mindestens 10 µm aufweist. Demgegenüber hat die äußere, erste Schicht in einem bevorzugten Ausführungsbeispiel eine vergleichsweise größere Schichtdicke, vorzugsweise eine Schichtdicke von mindestens 50 µm, besonders bevorzugt eine Schichtdicke von mindestens 80 µm, um eine ausreichende Stabilität des Katheterrohrelements zu gewährleisten. Anders ausgedrückt ist es von Vorteil, wenn das Verhältnis der Schichtdicke der ersten Schicht zur Schichtdicke der zweiten Schicht mindestens 3:1, vorzugsweise mindestens 4:1 beträgt.

In einem bevorzugten Ausführungsbeispiel sind die Schichtdicken der ersten und der zweiten Schicht über die gesamte Länge des Katheterrohrelements und den Querschnitt etwa gleich gestaltet. In weiteren Ausführungsbeispielen können die Schichtdicken der ersten und/oder der zweiten Schicht über die Länge des Katheterrohrelements bzw. den Querschnitt variieren.

Im Hinblick auf das Verfahren zur Herstellung eines derartigen Katheterrohrelements wird die oben angegebene Aufgabe durch ein Verfahren gelöst, bei dem in einem Schritt die erste Schicht und die zweite Schicht koextrudiert werden. Dieses Verfahren ist einfach und kostengünstig und erlaubt die Herstellung von Katheterrohrelementen in großtechnischem Maßstab.

Eine weitere Möglichkeit zur Herstellung eines derartigen Katheterrohrelements besteht darin, dieses mittels eines mehrstufigen Tauchverfahrens zu erzeugen. Hierfür wird eine, den inneren Hohlraum des Katheterrohrelements freihaltende Achse, die vorzugsweise zylinderförmig ist, bereitgestellt. Diese Achse wird anschließend zur Herstellung der ETFE-Schicht (zweite Schicht) einmal oder mehrmals in eine ETFE-Lösung getaucht, um die gewünschte Dicke der ETFE-Schicht zu erhalten. Anschließend wird die mit der ETFE-Schicht versehene Achse in eine Materiallösung getaucht, die das Material der ersten Schicht enthält. Auch dieser Tauchschritt kann gegebenenfalls mehrmals wiederholt werden, um die gewünschte Schichtdicke der ersten Schicht zu erhalten. Die Achse wird nach Beendigung dieses Schrittes entfernt.

Es zeigen schematisch:
- Fig. 1: eine perspektivische Seitenansicht eines Ausführungsbeispiels eines erfindungsgemäßen Katheterrohrelements und
- Fig. 2: eine Ansicht der Stirnseite des Ausführungsbeispiels gemäß Fig. 1.

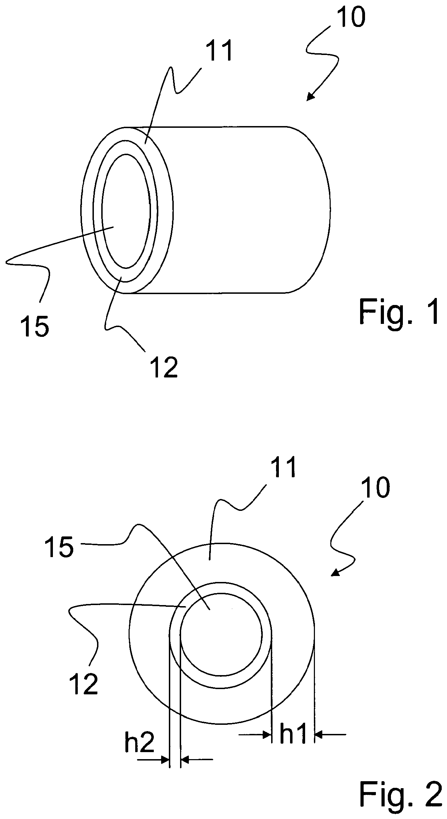

Das in Fig. 1 dargestellte Ausführungsbeispiel zeigt ein erfindungsgemäßes Katheterrohrelement 10, vorzugsweise für einen interventionellen Katheter, mit einer außen liegenden, ersten Schicht 11 und einer innen liegenden, zweiten Schicht 12. Das erfindungsgemäße Katheterrohrelement ist als Hohlzylinder mit einem in der durchgehenden Öffnung gebildeten inneren Lumen 15 gestaltet, das zur Anordnung und vorzugsweise zur Aufnahme eines nicht dargestellten Führungsdrahts bestimmt ist. Die zweite Schicht 12 bildet die gesamte Innenwand des Katheterrohrelements aus. Die erste Schicht 11 bildet die gesamte äußere Mantelfläche des Katheterrohrelements 10. Die erste Schicht 11 und die zweite Schicht 12 sind fest miteinander verbunden.

In einem weiteren Ausführungsbeispiel kann die zweite Schicht 12 auch lediglich teilweise, beispielsweise in einer Netz- oder Streifenstruktur, auf der Innenseite der ersten Schicht 11 angeordnet sein und deshalb die Innenwand des Katheterrohrelements nur teilweise ausbilden. In diesem Fall wird der nicht durch die zweite Schicht 12 gebildete Teil der Innenwand durch die unter der zweiten Schicht 12 angeordnete Schicht ausgebildet.

Die erste Schicht 11 enthält einen oder mehrere Kunststoffe aus der Gruppe PEBA, Polyamid und Elastomer modifiziertes Polyamid. Die zweite Schicht 12 enthält und besteht vollständig aus Ethylentetraflurethylen (ETFE). Die erste Schicht 11 besteht in einem bevorzugten Ausführungsbeispiel vollständig aus PEBA oder vollständig aus Polyamid oder vollständig aus einem Elastomer modifizierten Polyamid.

An einem distalen Ende des Katheterrohrelements kann ein nicht dargestelltes Ballonelement mit der ersten Schicht 11 verschweißt vorgesehen sein.

Die in Fig. 2 dargestellte Schichtdicke h1 der ersten Schicht beträgt mindestens 50 µm, vorzugsweise mindestens 80 µm. Die Schichtdicke h2 der zweiten Schicht 12 beträgt vorzugsweise wenige Mikrometer, bevorzugt mindestens 5 µm und besonders bevorzugt mindestens 10 µm. Das Verhältnis der Schichtdicken h1/h2 ist mindestens 3:1, vorzugsweise mindestens 4:1. Fig. 2 ist zu entnehmen, dass die erste Schicht 11 und die zweite Schicht 12 über den gesamten Querschnitt jeweils die gleiche Schichtdicke besitzen.

Mit der vorliegenden Erfindung wird ein Katheterrohrelement geschaffen, das einerseits strahlensterilisierbar und andererseits gute Biege- und Gleiteigenschaften besitzt. Außerdem ist die äußere Schicht des Katheterrohrelements gut schweißbar.

**Bezugszeichen:**

| | | | |
|---|---|---|---|
| 10 | Katheterrohrelement | h1 | Schichtdicke der ersten Schicht 11 |
| 11 | erste Schicht | h2 | Schichtdicke der zweiten Schicht 12 |
| 12 | zweite Schicht | | |
| 15 | inneres Lumen des Katheterrohrelements 10 | | |

## Patentansprüche

1. Katheterrohrelement (10), vorzugsweise zur Anordnung eines Führungsdrahtes, **dadurch gekennzeichnet, dass** das Katheterrohrelement besteht aus einer die äußere Schicht bildenden ersten Schicht (11) und einer mindestens teilweise die innere Schicht bildenden zweiten Schicht (12), wobei die erste Schicht mindestens einen Kunststoff oder mehrere Kunststoffe aus der Gruppe Polyether-Block-Amid (PEBA), Polyamid und Elastomer modifiziertes Polyamid enthält
**dadurch gekennzeichnet, dass** das Katheterrohrelement mittels Koextrusion oder mittels eines Tauchverfahrens, das vorzugsweise mehrstufig ist, hergestellt ist und dass die zweite Schicht (12) vollständig aus Ethylentetrafluorethylen besteht.

2. Katheterrohrelement nach Anspruch 1, **dadurch gekennzeichnet, dass** das Katheterrohrelement (10) hohlzylinderförmig ausgebildet ist.

3. Katheterrohrelement nach einem der Ansprüche 1 bis 2, **dadurch gekennzeichnet, dass** das Katheterrohrelement (10) aus genau zwei Schichten (11, 12) besteht.

4. Katheterrohrelement nach einem der vorhergehenden Ansprüche, **dadurch gekennzeichnet, dass** die zweite Schicht (12) eine Schichtdicke (h2) von 5 µm, bevorzugt eine Schichtdicke von mindestens 10 µm aufweist.

5. Katheterrohrelement nach einem der vorhergehenden Ansprüche, **dadurch gekennzeichnet, dass** die erste Schicht (11) eine Schichtdicke (h1) von mindestens 50 µm, vorzugsweise von mindestens 80 µm aufweist.

6. Katheterrohrelement nach einem der vorhergehenden Ansprüche, **dadurch gekennzeichnet, dass** das Verhältnis der Schichtdicke (h1) der ersten Schicht (11) zur Schichtdicke (h2) der zweiten Schicht (12) (h1 / h2) mindestens 3:1, vorzugsweise mindestens 4:1 beträgt.

7. Katheterrohrelement nach einem der vorhergehenden Ansprüche, **dadurch gekennzeichnet, dass** die Schichtdicke (h1) der ersten Schicht (11) und die Schichtdicke (h2) der zweiten Schicht (12) über die gesamte Länge des Katheterrohrelements und/oder den gesamten Querschnitt des Katheterrohrelements jeweils im Wesentlichen konstant bleibt.

8. Katheterrohrelement nach einem der vorhergehenden Ansprüche, wobei die erste Schicht (11) aus einem Material mit einem Härtegrad zwischen 45 bis 72 Shore D besteht.

## Claims

1. A catheter tube element (10), preferably for arranging a guide wire, **characterized in that** the catheter tube element consists of a first layer (11) forming an external layer, and a second layer (12) at least partially forming the internal layer, wherein the first layer comprises at least one plastic or multiple plastics from the group consisting of polyether block amide (PEBA), polyamide, and elastomer-modified polyamide, **characterized in that** the catheter tube element is produced by means of co-extrusion or by means of an immersion method, which preferably has a number of steps, and that the second layer (12) consists fully of ethylene tetrafluoroethylene.

2. The catheter tube element according to claim 1, **characterized in that** the catheter tube element (10) is hollow-cylindrical.

3. The catheter tube element according to either of claims 1 to 2, **characterized in that** the catheter tube element (10) consists of exactly two layers (11, 12).

4. The catheter tube element according to any one of the preceding claims, **characterized in that** the second layer (12) has a layer thickness (h2) of 5 µm, preferably a layer thickness of at least 10 µm.

5. The catheter tube element according to any one of the preceding claims, **characterized in that** the first layer (11) has a layer thickness (h1) of at least 50 µm, preferably a layer thickness of at least 80 µm.

6. The catheter tube element according to any one of the preceding claims, **characterized in that** the ratio of the layer thickness (h1) of the first layer (11) to the layer thickness (h2) of the second layer (12) (h1/h2) is at least 3:1, preferably at least 4:1.

7. The catheter tube element according to any one of the preceding claims, **characterized in that** the layer thickness (h1) of the first layer (11) and the layer thickness (h2) of the second layer (12) each remain essentially constant over the entire length of the catheter tube element and/or the entire cross-section of the catheter tube element.

8. The catheter tube element according to any one of the preceding claims, wherein the first layer (11) consists of a material having a degree of hardness between 45 and 72 Shore D.

## Revendications

1. Elément de tube de cathéter (10), préférentiellement destiné à mettre en place un fil de guidage, **caractérisé en ce que** ledit élément de tube de cathéter est constitué d'une première couche (11) formant la couche extérieure et d'une deuxième couche (12) formant au moins partiellement la couche intérieure, la première couche contenant au moins une matière plastique ou plusieurs matières plastiques issues du groupe polyéther à blocs d'amide (PEBA), polyamide et polyamide modifié aux élastomères
**caractérisé en ce que** l'élément de tube de cathéter est fabriqué par coextrusion ou par un processus d'immersion comportant de préférence plusieurs étapes et que la deuxième couche (12) est entièrement constituée d'éthylène-tétrafluoroéthylène.

2. Elément de tube de cathéter selon la revendication 1, **caractérisé en ce que** l'élément de tube de cathéter (10) est réalisé sous forme d'un cylindre creux.

3. Elément de tube de cathéter selon l'une des revendications 1 à 2, **caractérisé en ce que** l'élément de tube de cathéter (10) est constitué d'exactement deux couches (11, 12).

4. Elément de tube de cathéter selon l'une des revendications précédentes, **caractérisé en ce que** la deuxième couche (12) présente une épaisseur de couche (h2) de 5 µm, préférentiellement une épaisseur de couche d'au moins 10 µm.

5. Elément de tube de cathéter selon l'une des revendications précédentes, **caractérisé en ce que** la première couche (11) présente une épaisseur de couche (h1) d'au moins 50 µm, préférentiellement d'au moins 80 µm.

6. Elément de tube de cathéter selon l'une des revendications précédentes, **caractérisé en ce que** le rapport entre l'épaisseur de couche (h1) de la première couche (11) et l'épaisseur de couche (h2) de la deuxième couche (12) (h1 / h2) est supérieur ou égal à 3 : 1, de préférence supérieur ou égal à 4 : 1.

7. Elément de tube de cathéter selon l'une des revendications précédentes, **caractérisé en ce que** l'épaisseur de couche (h1) de la première couche (11) et l'épaisseur de couche de (h2) de la deuxième couche (12) restent sensiblement constantes sur toute la longueur de l'élément de tube de cathéter et/ou toute la coupe transversale de l'élément de tube de cathéter.

8. Elément de tube de cathéter selon l'une des revendications précédentes, la première couche (11) étant constituée d'un matériau dont le degré de dureté est compris entre 45 et 72 Shore D.
